Europäisches Patentamt

European Patent Office

Office européen des brevets

(11) Publication number: **0 209 490 B1**

(12) **EUROPEAN PATENT SPECIFICATION**

(45) Date of publication of patent specification: **14.10.92** (51) Int. Cl.5: **G01N 33/53**, //G01N33/553

(21) Application number: **86810292.2**

(22) Date of filing: **03.07.86**

(54) **Container for determining the quantity of antibodies or antigens in a biological liquid.**

(30) Priority: **08.07.85 CH 2956/85**

(43) Date of publication of application:
**21.01.87 Bulletin 87/04**

(45) Publication of the grant of the patent:
**14.10.92 Bulletin 92/42**

(84) Designated Contracting States:
**AT BE CH DE FR GB IT LI LU NL SE**

(56) References cited:
**FR-A- 2 423 769**
**FR-A- 2 454 098**
**GB-A- 1 478 620**
**US-A- 4 106 907**
**US-A- 4 229 104**

(73) Proprietor: **Applied Research Systems ARS Holding N.V.**
**6 John B. Gorsiraweg**
**Curaçao(AN)**

(72) Inventor: **Ringrose, Anthony**
**124 Chemin de la Montagne**
**CH-1224 Chenes-Bougeries/Geneva(CH)**

(74) Representative: **Dousse, Blasco et al**
**7, route de Drize**
**CH-1227 Carouge/Genève(CH)**

## Description

The present invention concerns a container for determining the quantity of antibodies or antigens in a biological liquid by creating specific antibody-antigen complexes, one of said antigen or antibody being initially associated with suspended magnetic particles, said complexes being separated from the remainder of a liquid sample by gathering them against a side wall of the container by magnetic attraction of said particles.

A method of adding antibodies or antigens to a biological liquid is known. It consists of suspending in the liquid magnetic particles associated with either antigens or antibodies specific to the respective antibodies or antigens that are to be ascertained in sufficient excess to ensure that all the antibodies or antigens to be measured will combine with a respective antigen or antibody specific to them. The magnetic particles are then attracted to one wall of the container, all substances except the magnetic particles associated with their specific antigen or antibody reactant combined or uncombined with their respective antibody or antigen partner are evacuated from the container, the container is rinsed out, a reaction liquid is introduced to create an enzymatic reaction with the antibodies or antigens, the particles are suspended in the liquid for a prescribed period, an agent is added to the liquid to terminate the reaction, the magnetic particles are again attracted to the one wall of the container, and the reaction liquid is measured colorimetrically.

Attracting the magnetic particles to the bottom of a dish and evacuating the biological liquid by turning the dish upside down along with the magnet, which would then maintain the particles against the bottom, has already been proposed. This procedure, however, is not very practical. Turning a dish upside down inside automatic analysis equipment necessitates a large number of mechanisms. It is also necessary to make provisions for collecting the liquid that spills out of the dishes.

One variation of the proposed method involves positioning the magnet at one side of the dish to attract the magnetic particles to a certain area of the wall. The bottom of the dish accordingly consists of a cavity, either in the form of a spherical cap tangent to a tubular body in the case of a dish with a circular cross-section or by a cavity with a straight generatrix parallel to two opposing faces of the tube and tangent to the faces in the case of a dish with a rectangular cross-section. The cavities that are tangent to the tubular section of the dish are intended to promote stirring the liquid and prevent dead zones in which it will remain stationary during agitation, making the mixture non-homogeneous. The drawback to a dish bottom of this particular shape is that the magnet that attracts the magnetic particles to a certain arrea of the wall has to be constantly retained at that area in order to maintain them against the wall. This implies in turn that, in automatic analysis equipment in which the dishes are transported from one operating point to another by a conveyor, the magnet must be part of the conveyor, requiring one magnet for each dish as well as means of shifting the magnet between an active and inactive position. Such a solution is complicated and hence expensive. If the magnets are positioned only along the sections of the path of the conveyor within which the magnetic particles are to be attracted to the particular portion of the wall of the dish, the particles will slide farther along the wall every time the conveyor shifts the dish from one magnet to the next and will eventually arrive more or less at the bottom of the dish. In as much as the whole method of collecting the magnetic particles within a certain area of the wall of the dish is intended to allow evacuation of the liquid with a pipette, some of the particles that slide down to the bottom will also be evacuated and will inadmissibly contaminate the measurement results.

It has already been proposed to form liquid sample tubes with special configurations in order to confer specific features to these tubes. However, none of the prior art sample tubes has been designed to maintain magnetic particles against a certain area of a lateral wall of a dish.

A cuvette which comprises tapered inner surfaces which make a funnel-shaped transition into its lower portion is disclosed in US-A 4,229,104. The slope of these tapered inner surfaces would be to steep in order to maintain magnetic particles against the upright lateral face of this cuvette. A similar tube is also disclosed in US-A 4,106,907. Another liquid sample cup is disclosed in GB-A 1,478,620, which has parallel side walls, a rear wall and a forward wall whilst being parallel to the rear wall for a short distance from the top of the cup, then inclines downward toward the rear wall to form a lower closed portion which is offset from the centreline of the cup. The inclined face makes an angle of more than 140° with the forward wall, what is to steep to maintain magnetic particles against the forward wall. Moreover a curved portion connects the forward wall to the inclined face which would still promote the sliding of the magnetic particles toward the bottom.

A plate for biological agglutination tests is disclosed in FR-A 2,423,769. This plate is integral with eight rows of twelve cells surrounded by a rim, so that it is not possible to use these cells separately. Moreover the rim which surrounds the cells does not allow to selectively place a permanent magnet closely to the side wall of the cells which are disposed in the peripheral rows of cells. These pheripheral rows and the rim should no longer allow to come laterally close to the other cells with a permanent magnet.

The object of the present invention is to at least to some extent eliminate the drawbacks of the aforesaid methods.

This object is attained in accordance with the invention in a container for determining the quantity of antibodies or antigens as recited in Claim 1.

The advantage of this container is that the surface at an obtuse angle to one lateral face of the dish briefly prevents the collected magnetic particles from sliding. When the magnetic particles collect against the lateral face of the container that is adjacent to the obtuse angle and within the area of the face that is adjacent to the apex of the angle, although briefly released from the effect of the magnetic field that has collected them, the particles are actually prevented from sliding down due to the oblique face at an obtuse to the lateral face that they collect against. Furthermore, since the oblique face is tangent to a concave area that is itself tangent to the opposite lateral face, the shape of the bottom of the container will not allow dead zones while the liquid is being mixed because the shape will essentially coincide with the rounded shape of the dish. A dish bottom of this shape will allow pipetting below the level at which the particles are collected, and both the biological liquid and the rinse can be evacuated from the container.

One embodiment and a variant of the invention will now be described with reference to the attached drawing, in which

Figure 1 is a front view of a container in accordance with the invention and

Figure 2 is a section along the line II-II in Figure 1.

Figure 3 is a section of the variant along a diameter thereof.

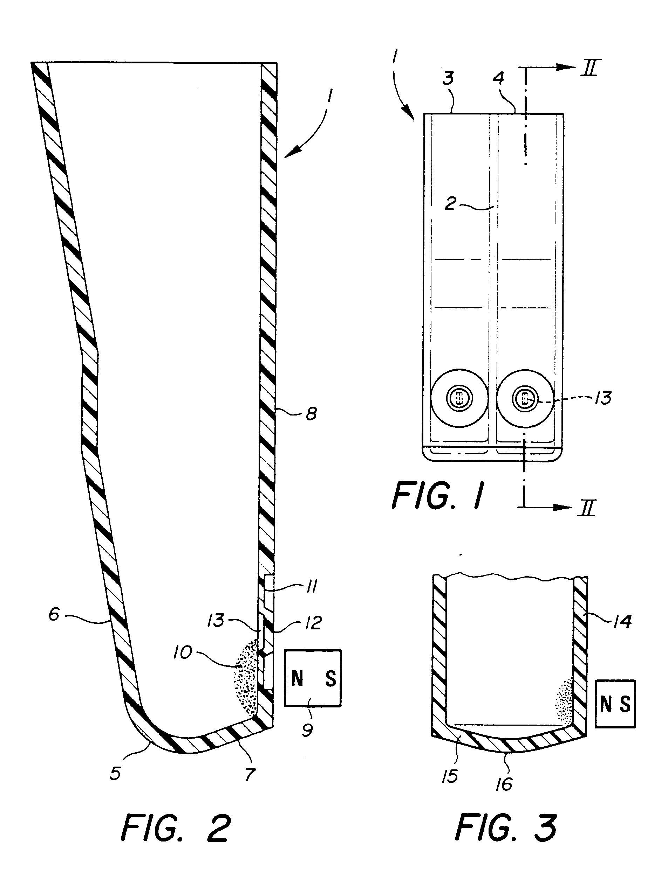

The illustrated embodiment of a container in accordance with the invention (see figures 1 and 2) consists of a tubular element 1 divided into two compartments 3 and 4 that are rectangular in cross-section and taper slightly out and up by a partition 2. One compartment is intended to accomodate a sample to be analyzed along with its appropriate reagent or reagents and the other to accomodate a control substance that functions as a reference for the particular test being conducted along with its appropriate reagent or reagents. The invention is obviously not restricted to an embodiment employing only two compartments.

It will be evident from Figure 2 that the section of the bottom of the container is not an arc of a circle tangent to two opposing lateral faces as in all other analysis containers of this type. The actual shape is intended to prevent the establishment of dead zones while the liquid is being agitated. The cross-section of the bottom is on the other hand an arc 5 of a circle that, while it is tangent to one lateral face 6 of the container, is not tangent to the opposite lateral face but to an oblique face 7 that is itself at an obtuse angle to the opposite lateral face 8.

The area of opposite lateral face 8 that is adjacent to the obtuse angle with oblique face 7 is intended to be positioned opposite a permanent magnet 9. The magnet collects the magnetic particles 10 suspended within compartments 3 and 4 for the purpose specified previously herein. The particular obtuse angle between faces 7 and 8 in the illustrated embodiment is 110°. The closer the angle is to 90° the more the particles will be retained against the base of opposite lateral face 8, although, if the angle were exactly 90°, the bottom of the container and the lower part of the area that the particles collect in would be at the same level. Furthermore, the closer the angle is to 90° the greater the risk of a dead zone while the liquid is being agitated. It is, rather, preferable as previously discussed herein, for the point of the pipette to arrive below the lower part of the area that the particles collect in. In practice, the angle between faces 7 and 8 is between 105 and 120°.

The area of the lateral face 8 that magnetic particles 10 are supposed to collect in approximately coincides with an area of the wall of each compartment 3 and 4 that is provided with a depressed annular surface 11 (Fig. 2) surrounding a circular area 12 as thick as the rest of the wall. Depressed annular surface 11 is entended as a sort of a flexible diaphragm that, although it is in one piece with the wall of the container, can move elastically in relation to the rest of the wall. There is a groove 13 in the center of the inside of circular area 12. Annular surface 11, circular area 12, and groove 13 are intended to transmit to the liquid the ultrasonic vibrations generated by a transducer positioned against circular area 12 in order to agitate the liquid in consequence of the cavitation thereby induced in it. It should be emphasized that the annular surface 11 is located in the vicinity of the apex of the angle between faces 7 and 8. This location is preferable in that it also helps prevent the establishment of dead zones in the only region of the liquid where the agitation could possibly be attenuated. Furthermore, once the particles have been collected in the area of wall 8 adjacent to the obtuse angle and when they are to be resuspended, the ultrasound will be able to act directly on them and disperse them.

The outside diameter of the annular surface 11 in the illustrated embodiment is 6 mm and its inside diameter 2 mm.

The wall of the container is 0.8 mm thick and the annular surface 0.3 mm thick. The container is extruded of polystyrene or polyacrylate.

Although the compartments 3 and 4 in the illustrated embodiment are in the shape of tubes with a rectangular cross-section it will be obvious that the invention can also be unrestrictedly applied to tubes with a different cross-section, circular for example. The rectangular shape, however, will permit colorimetric measurements of the liquid, which is facilitated when the beam can pass through parallel surfaces.

Figure 3 illustrates a variant in which a tubular enclosure is used. In this variant, the enclosure bottom consists of a frustoconical portion 15 whose bevel angle is between 105 and 120°. This frustoconical portion is terminated by a spherical cap 16 for ensuring a complete aspiration of the liquid phase, which operation would be more difficult if the bottom were conically shaped.

## Claims

1. Container for determining the quantity of antibodies or antigens in a biological liquid by forming specific antibody-antigen complexes, one of said antigen or antibody being initially associated with suspended magnetic particles, said complexes being separated from the remainder of a liquid sample by gathering them against a side wall (8) of the container by magnetic attraction of said particles, characterized in that it comprises a tubular body having an open upper end and a lower end closed by a bottom wall; said tubular body having a side wall at a lower part of which is a collecting surface portion located above said bottom wall and directly laterally accessible from the space surrounding said tubular body whereby a magnet may be placed adjacent said collecting surface; said bottom wall having a cross section which includes a curved portion (5,16) defining an arc of a circle and a retaining surface portion (7,15) located between said curved portion of said bottom wall and said collecting surface portion of said wall, said retaining surface portion (7,15) being substantially planar and forming an angle with said collecting surface portion of substantially 105° to 120°.

2. Container as in claim 1, characterized in that it has a rectangular cross-section, said surface (7) generated by a straight generating line being planar and adjacent to one of the side walls of the container and at a tangent to a concave surface which is itself at a tangent to the opposite side wall of the container.

3. Container as in claim 2, characterized by a partition (2) extending between two opposite side walls and dividing the container into two identical compartments.

4. Container as in claim 1, characterized in that said portion of the side wall (8) through which the magnetic field is intended to extend includes a region for transmitting ultrasound and consisting of an area (12) that is less rigid than the rest of the wall and that is elastically connected (11) at its periphery to the rest of the wall.

## Patentansprüche

1. Behälter zur Bestimmung der Menge von Antikörpern der Antigenen in einer biologischen Flüssigkeit durch Ausbildung von spezifischen Antikörper-Antigen Komplexen, wobei die einen, Antigene oder Antikörper, anfänglich mit fein verteilten magnetischen Teilchen verbunden sind und die Komplexe vom Rest der Flüssigkeitsprobe getrennt werden, indem sie gegen eine Seitenwand (8) des Behälters durch magnetische Anziehung der Teilchen angesammelt werden, dadurch gekennzeichnet, daß er einen rohrförmigen Körper aufweist, der ein oberes, offenes Ende hat und ein unteres Ende, das durch eine Bodenwand geschlossen ist; der rohrförmige Körper hat eine Seitenwand, an deren unterem Teil ein Sammelflächenabschnitt angeordnet ist, der oberhalb der Bodenwand liegt und direkt seitlich über den Raum zugänglich ist, der den rohrförmigen Körper umgibt, wobei ein Magnet angrenzend an diese Sammelfläche angeordnet werden kann; die Bodenwand hat einen Querschnitt, welcher einen gekrümmten Abschnitt (5, 16), der einen Kreisbogen beschreibt und einen verbleibenden Flächenabschnitt (7, 15), der zwischen dem gekrümmten Abschnitt der Bodenwand und dem Sammelflächenabschnitt angeordnet ist, enthält, dabei ist der verbleibende Abschnitt (7, 15) im wesentlichen eben und bildet mit dem Samelflächenabschnitt einen Winkel von im wesentlichen 105° bis 120°.

2. Behälter gemäß Anspruch 1, dadurch gekennzeichnet, daß er einen rechteckigen Querschnitt hat, wobei diese Fläche (7), die durch eine gerade Zeugelinie erzeugt wird, eben und an eine der Seitenwände des Behälters angrenzend und zu einer konkaven Fläche tangential ist, die selbst tangential zur gegenüberliegenden Seitenwand des Behälters ist.

3. Behälter gemäß Anspruch 2, gekennzeichnet durch eine Zwischenwand (2), die sich zwischen den beiden gegenüberliegenden Seitenwänden erstreckt und den Behälter in zwei identische Abteilungen trennt.

**4.** Behälter gemäß Anspruch 1, dadurch gekennzeichnet, daß der Abschnitt der Seitenwand (8), durch welchen das Magnetfeld sich erstrecken soll, einen Bereich zur Übertragung von Ultraschall enthält und aus einer Fläche (12) besteht, die weniger fest als der Rest der Wand ist und die an ihrem Rand mit dem Rest der Wand elastisch verbunden (11) ist.

**Revendications**

**1.** Enceinte pour déterminer la quantité d'anticorps ou d'antigénes dans un liquide biologique par la formation de complexes anticorps-antigène spécifiques, un desdits antigènes ou anticorps étant initialement associé à des particules magnétiques en suspension, ces complexes étant séparés du reste d'un échantillon liquide en les rassemblant contre une paroi latérale (8) de l'enceinte par attraction magnétique de ces particules, caractérisée en ce qu'elle comporte un corps tubulaire présentant une extrémité supérieure ouverte et une extrémité inférieure fermée par un fond; ce corps tubulaire présentant une paroi latérale à une partie inférieure de laquelle se trouve une portion de surface collectrice située au-dessus de ce fond et accessible latéralement directement depuis l'espace entourant ce corps tubulaire de sorte qu'un aimant peut être placé adjacent à cette surface collectrice; ce fond présentant une section transversale qui comporte une portion arquée (5,16) définissant un arc de cercle et une portion de surface de retenue (7,15) située entre cette portion arquée du fond et la portion de surface collectrice de ladite paroi, la portion de surface de retenue (7,15) étant pratiquement plane et formant un angle avec la portion de surface collectrice de pratiquement 105° à 120°.

**2.** Enceinte selon la revendication 1, caractérisée en ce qu'elle présente une section transversale rectangulaire, ladite surface (7) engendrée par une génératrice droite étant plane et adjacente à l'une des parois latérales de l'enceinte et tangente à une surface concave qui est elle-même tangente à une paroi latérale opposée de l'enceinte.

**3.** Enceinte selon la revendication 2, caractérisée par une cloison (2) s'étendant entre deux parois latérales opposées et divisant l'enceinte en deux compartiments identiques.

**4.** Enceinte selon la revendication 1, caractérisée en ce que ladite portion de la paroi latérale (8) à travers laquelle le champ magnétique est destiné à s'étendre comporte une zone pour transmettre des ultra-sons et comprenant une zone (12) qui est moins rigide que le reste de la paroi et qui est reliée élastiquement (11) à sa périphérie au reste de la paroi.

FIG. I

FIG. 2

FIG. 3

N S

N S